# EUROPEAN PATENT APPLICATION

(11) **EP 4 663 639 A1**
(43) Date of publication of application: **17.12.2025**
(21) Application number: 24752803.7
(22) Date of filing: 02.02.2024
(51) Int. Cl.: C07D 471/22, A61K 31/438, A61K 31/4375, A61P 29/00, A61P 25/00

(54) **SALT AND CRYSTAL OF MNK INHIBITOR COMPOUND AND PREPARATION METHOD THEREFOR**

(30) Priority: 10.02.2023 CN 202310097521
(71) Applicant: Novostar Pharmaceuticals, Ltd., Shanghai 201210 (CN)
(72) Inventor: LI, Bing, Shanghai 201210 (CN); ZHANG, Ke, Shanghai 201210 (CN)
(74) Representative: EIP
(86) International application number: PCT/CN2024/075454
(87) International publication number: WO 2024/164947

(57) **Abstract**

A salt of a compound as an MNK inhibitor, in particular, a sulfate of the compound, and a crystal of the compound, and a preparation method therefor.

## Description

### Technical Fields

The present invention relates to a salt of a compound as an MNK inhibitor, in particular, a sulfate of the compound, and a crystal of the compound, and a preparation method therefor.

### Background Technology

In recent years, it has been found that the expression of serine/threonine-protein kinase (MNK) has a strong correlation with proliferative diseases, inflammatory diseases and neurodegenerative diseases. For example, WO2020155842A1 discloses a class of MNK inhibitors that have potential therapeutic effects in the treatment of a variety of conditions such as proliferative diseases, inflammatory diseases, and neurodegenerative diseases, and can be used to prepare pharmaceutical compositions for the treatment of diseases or conditions associated with MNK activity or expression.

As a preferred compound among such MNK inhibitors, it is known to have the Chemical Formula 1 "N-(12'-methyl-1',5'-dioxo-1',5',6 ',11'-tetrahydro-2'H-spiro[cyclohexane-1,3'-imidazo[1,5':1,6]pyrido[3,4-b][ 1,6]naphthyridin]-8'-yl)cyclopropane carboxamide" has unique and highly efficient antitumor activity and immunomodulatory effects, and has shown excellent antitumor activity in a variety of hematological and solid tumor models, and is a broad-spectrum anticancer drug with great potential. However, the chemical stability of this compound is relatively low, rendering it susceptible to oxidation or aromatization under ambient temperature, atmospheric pressure or light exposure. In addition, the low solubility of this compound affects the concentration of the active component, thereby rendering the conditions for its production and storage rather stringent.

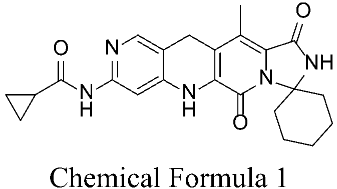

Therefore, when used as an active ingredient in pharmaceutical compositions, it needs to be converted into a more structurally stable form, thereby enabling the concentration of the active ingredient to be maintained for a long period of time and reducing the requirements for process conditions, so that industrialization can be achieved under relatively mild conditions.

### Invention Content

The object of the present invention is to provide an "N-(12'-methyl-1',5"-dioxo-1",5",6',11'-Tetrahydro-2'H-spiro[cyclohexane-1, 3'-imidazo[1,5':1,6]pyrido[3,4-b][1,6]naphthyridine]-8'-yl)cyclopropane carboxamide" (hereinafter referred to as "Compound 1") salts and its crystal forms.

In a first aspect of the present invention, there is provided a salt of a compound of Chemical Formula 1:

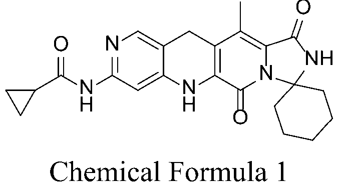

The salt comprises a free base moiety and an acid moiety.

The free base moiety has the structure of Chemical Formula 1.

The acid moiety is any one selected from hydrochloric acid, sulfuric acid, methanesulfonic acid or formic acid.

By converting the compound into a salt, it is possible to improve its solubility properties in solvents and to improve its stability. For example, when the acid moiety is an inorganic or organic acid, such that the salt of the compound exhibits enhanced solubility in water and/or organic solvents, reduced propensity for oxidation or aromatization, and improved storage stability. The above effects are particularly pronounced when the acid moiety is any one selected from hydrochloric acid, sulfuric acid, methanesulfonic acid, or formic acid.

In preferred embodiments, the salt is in a crystalline form.

In a preferred embodiment, the acid moiety is sulfuric acid.

The inventor found through extensive experimental studies that when the acid moiety of the salt is sulfuric acid, the resulting salt exhibits optimal chemical and storage stability, better water solubility and lower hygroscopicity; at the same time, the preparation process of the salt is simple and reproducible. Thus, it is considered that the salt with the acid moiety of sulfuric acid possesses significant pharmaceutical developability potential.

In a second aspect of the present invention, there is provided a crystal of sulfate salt of a compound of Chemical Formula 1:
the crystal has a crystal structure of polymorph A, the X-ray powder diffraction pattern of the polymorph A exhibits characteristic peaks at 2θ values of 7.44±0.2°, 15.72±0.2°, 20.05±0.2°, 24.10±0.2°, 24.57±0.2°, 25.55±0.2° and 27.10±0.2°.

In a preferred embodiment, the X-ray powder diffraction pattern of the polymorph A also has one or more characteristic peaks at 2θ values selected from 12.33±0.2°, 13.50±0.2°, 14.83±0.2°, 18.33±0.2°, 19.70±0.2°, 21.30±0.2° and 29.77±0.2°.

In the preferred embodiment, the X-ray powder diffraction pattern of the polymorph A exhibits characteristic peaks at 2θ values of 7.44±0.2°, 10.52±0.2°, 12.33±0.2°, 13.50±0.2°, 14.83±0.2°, 15.72±0.2°, 17.43±0.2°, 18.33±0.2°, 19.70±0.2°, 20.05±0.2°, 21.30±0.2°, 24.10±0.2°, 24.57±0.2°, 25.55±0.2°, 27.10±0.2°, 27.93±0.2°, and 29.77±0.2°.

In the preferred embodiment, the X-ray powder diffraction pattern of the polymorph A exhibits characteristic peaks at 2θ values of 7.44±0.2°, 10.52±0.2°, 11.23±0.2°, 12.33±0.2°, 13.50±0.2°, 14.83±0.2°, 15.34±0.2°, 15.72±0.2°, 17.43±0.2°, 18.33±0.2°, 18.81±0.2°, 19.70±0.2°, 20.05±0.2°, 21.30±0.2°, 22.31±0.2°, 23.20±0.2°, 24.10±0.2°, 24.57±0.2°, 25.05±0.2°, 25.55±0.2°, 26.04±0.2 °, 27.10±0.2°, 27.93±0.2°, 29.77±0.2°, 30.33±0.2°, 30.99±0.2°, 32.10±0.2°, 32.75±0.2°, 33.69±0.2°, 34.83±0.2°, and 35.83±0.2°.

In preferred embodiments, the crystal of sulfate salt is a hydrate, in particular a dihydrate.

In a third aspect of the present invention, there is provided a method of preparing the crystal of a sulfate salt of a compound of Chemical Formula 1, which comprises the steps of: mixing the compound of Chemical Formula 1 with a solvent selected from water, alcohols, ketones, or mixtures thereof, and adding a sulfuric acid solution to form a suspension; stirring the suspension and then separating it to obtain crystals.

In a preferred embodiment, the solvent is a mixture of water and ketones.

In a preferred embodiment, the solvent is a mixture of water and acetone.

In the preferred embodiment, the volume ratio of water to acetone is 1/99 to 30/70.

In the preferred embodiment, the volume ratio of water to acetone is 5/95 to 10/90.

In the preferred embodiment, 1.1 to 2.1 equivalents of sulfuric acid solution is added.

In a fourth aspect of the present invention, there is provided a method of preparing the crystal of a sulfate salt of a compound of Chemical Formula 1, comprising: mixing the compound of Chemical Formula 1 with a solvent selected from water, alcohols, ketones, or mixtures thereof, and forming a suspension by adding a sulfuric acid solution; adding a sulfate salt polymorph A of the compound of Chemical Formula 1 as a seed crystal to the suspension; stirring the suspension and then isolating crystals by separation.

In preferred embodiments, the solvent is selected from water, methanol, ethanol, n-propanol, isopropanol, 1-butanol, tert-butanol, acetone, butanone, 2-pentanone or mixtures thereof.

In preferred embodiments, the solvent is selected from water, ethanol, acetone or mixtures thereof.

In a fifth aspect of the present invention, there are provided pharmaceutical compositions comprising a salt as described in the first aspect or a crystal as described in the second aspect, and a pharmaceutically acceptable excipient.

The conversion of the compound of Chemical Formula 1 into a salt form result in enhanced structural stability, so that it is not susceptible to oxidization or aromatization under certain humidity and room temperature environmental conditions, which would otherwise convert it into other components and thereby reduce the content of the effective active component.

The more stable structure of the salt reduces the requirements for production and storage process conditions, making it easier to be processed into drugs and industrialized.

The sulfate crystals of the compound of Chemical Formula 1 prepared by the method of the present invention are characterized by optimal chemical and storage stability, better water solubility and lower hygroscopicity, etc., and the preparation process is simple and controllable, with good reproducibility, which endows this component with significant pharmaceutical developability potential.

It should be understood that, within the scope of the present invention, each of the above technical features of the present invention and each of the technical features specifically described hereinafter (e.g., embodiments) may be combined with each other, thereby constituting a new or preferred technical solution. For the sake of space, we will not repeat them herein.

### The Accompanying Illustration

Figure 1 shows the X-ray powder diffraction (XRPD) pattern of polymorph I of compound **1** obtained in Comparative Example 1;
Figure 2 shows the X-ray powder diffraction (XRPD) pattern of the polymorph A of sulfate salt of compound **1** obtained in Example 1;
Figure 3 shows the thermogravimetric analysis (TGA) profile of the polymorph A of sulfate salt of compound **1** obtained in Example 1;
Figure 4 shows a differential scanning calorimetry (DSC) profile of the polymorph A of sulfate salt of compound **1** obtained in Example 1;
Figure 5 shows the X-ray powder diffraction (XRPD) pattern of the polymorph B of hydrochloride salt of compound **1** obtained in Example 2;
Figure 6 shows the X-ray powder diffraction (XRPD) pattern of the polymorph B of mesylate salt of compound **1** obtained in Example 3;
Figure 7 shows the X-ray powder diffraction (XRPD) pattern of the polymorph A of formate salt of compound **1** obtained in Example 4;
Figure 8 shows the X-ray powder diffraction (XRPD) pattern of the polymorph B of sulfate salt of compound **1** obtained in Example 7;
Figure 9 shows the X-ray powder diffraction (XRPD) pattern of the polymorph C of sulfate salt of compound **1** obtained in Example 8;
Figure 10 shows the X-ray powder diffraction (XRPD) pattern of the polymorph D of sulfate salt of compound **1** obtained in Example 9;
Figure 11 shows the X-ray powder diffraction (XRPD) pattern of the polymorph E of sulfate salt of compound **1** obtained in Example 10;
Figure 12 shows the X-ray powder diffraction (XRPD) pattern of the polymorph F of sulfate salt of compound **1** obtained in Example 11;
Figure 13 shows the X-ray powder diffraction (XRPD) pattern of the polymorph G of sulfate salt of compound **1** obtained in Example 12;
Figure 14 shows a comparative X-ray powder diffraction diagram of polymorph A of sulfate salt of compound **1** obtained in Example 1 after being placed for 1 week at different temperatures and humidity conditions;
Figure 15 shows a comparative X-ray powder diffraction diagram of the polymorph A of sulfate salt of compound **1** obtained in Example 1 after being left for 4 weeks at different temperatures and humidity conditions;
Figure 16 shows the dynamic water sorption (DVS) profile of polymorph A of sulfate salt of compound **1** obtained in Example 1.

### Detailed Description of the Embodiments

The present invention is further elaborated in the following with specific embodiments. It should be understood that the detailed description of the technical solution of the present invention through the following embodiments will facilitate a further understanding of the advantages and effects of the technical solution of the present invention. The embodiments do not limit the scope of protection of the present invention, which is defined by the claims.

In a first aspect of the present invention, there is provided a salt of a compound of Chemical Formula 1:

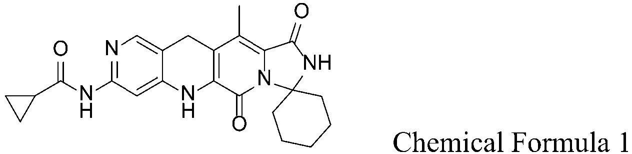

The salt comprises a free base moiety and an acid moiety.

The free base moiety has the structure of Chemical Formula 1.

The acid moiety is any one selected from hydrochloric acid, sulfuric acid, methanesulfonic acid or formic acid.

By converting the compound into a salt, it is possible to improve its solubility properties in solvents and to improve its stability. For example, when the acid moiety is an inorganic or organic acid, such that the salt of the compound exhibits enhanced solubility in water and/or organic solvents, reduced propensity for oxidation or aromatization, and improved storage stability. The above effects are particularly pronounced when the acid moiety is any one selected from hydrochloric acid, sulfuric acid, methanesulfonic acid, or formic acid.

In preferred embodiments, the salt is in a crystalline form.

It is known that the free base form of compound **1** or its amorphous salt is susceptible to oxidation or aromatization under ambient temperature and pressure, which affects the chemical structure of the active component and consequently impairs its pharmaceutical properties. Compound **1** in the form of a crystalline salt exhibits higher chemical stability than its free base or amorphous salt form.

In a preferred embodiment, the acid moiety is sulfuric acid.

The inventor found through extensive experimental studies that when the acid moiety of the salt is sulfuric acid, the resulting salt exhibits optimal chemical and storage stability, better water solubility and lower hygroscopicity; at the same time, the preparation process of the salt is simple and reproducible. Thus, it is considered that the salt with the acid moiety of sulfuric acid possesses significant pharmaceutical developability potential.

In a second aspect of the present invention, there is provided a crystal of sulfate salt of a compound of Chemical Formula 1:
The crystal has a crystal structure of polymorph A, the X-ray powder diffraction pattern of the polymorph A exhibits characteristic peaks at 2θ values of 7.44±0.2°, 15.72±0.2°, 20.05±0.2°, 24.10±0.2°, 24.57±0.2°, 25.55±0.2° and 27.10±0.2°.

In a preferred embodiment, the X-ray powder diffraction pattern of the polymorph A also has one or more characteristic peaks at 2θ values selected from: 12.33±0.2°, 13.50±0.2°, 14.83±0.2°, 18.33±0.2°, 19.70±0.2°, 21.30±0.2° and 29.77±0.2°.

In the preferred embodiment, the X-ray powder diffraction pattern of the polymorph A exhibits characteristic peaks at 2θ values of 7.44±0.2°, 10.52±0.2°, 12.33±0.2°, 13.50±0.2°, 14.83±0.2°, 15.72±0.2°, 17.43±0.2°, 18.33±0.2°, 19.70±0.2°, 20.05±0.2°, 21.30±0.2°, 24.10±0.2°, 24.57±0.2°, 25.55±0.2°, 27.10±0.2°, 27.93±0.2°, and 29.77±0.2°.

In the preferred embodiment, the X-ray powder diffraction pattern of the polymorph A exhibits characteristic peaks at 2θ values of 7.44±0.2°, 10.52±0.2°, 11.23±0.2°, 12.33±0.2°, 13.50±0.2°, 14.83±0.2°, 15.34±0.2°, 15.72±0.2°, 17.43±0.2°, 18.33±0.2°, 18.81±0.2°, 19.70±0.2°, 20.05±0.2°, 21.30±0.2°, 22.31±0.2°, 23.20±0.2°, 24.10±0.2°, 24.57±0.2°, 25.05±0.2°, 25.55±0.2°, 26.04±0.2 °, 27.10±0.2°, 27.93±0.2°, 29.77±0.2°, 30.33±0.2°, 30.99±0.2°, 32.10±0.2°, 32.75±0.2°, 33.69±0.2°, 34.83±0.2°, and 35.83±0.2°.

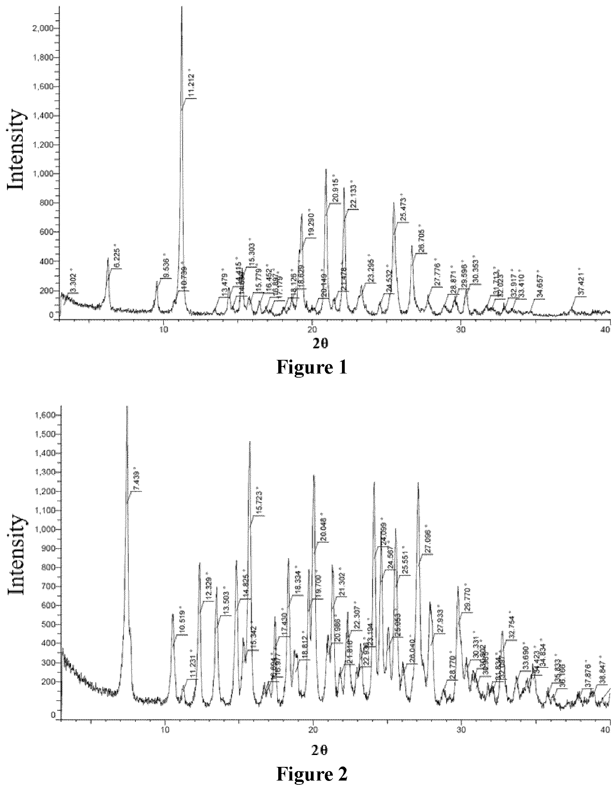

The X-ray powder diffraction patterns of polymorph A are slightly different by the nuances of the crystal structure, which are mainly influenced by the specific crystallization conditions. In a specific embodiment, the X-ray powder diffraction pattern of the polymorph A is shown in FIG. 2.

In preferred embodiments, the crystal of sulfate salt is a hydrate, in particular a dihydrate.

As a component of a pharmaceutical composition, it is usually required that residual organic solvents be minimized. When there is a solvent compound structure in the component, desolvation may occur under changes in temperature or pressure. Thus, when the solvent is an organic solvent, the released solvent may cause safety problems in the use of the drug. When the solvent is water, even if dehydration occurs, the safety of the drug can still be guaranteed.

In some embodiments, the salt has about 1.5 equivalents of hydrate relative to the salt; in some embodiments, the salt has about 2 equivalents of hydrate relative to the salt; in some embodiments, the salt has about 2.5 equivalents of hydrate relative to the salt; in some embodiments, the salt has about 3 equivalents of hydrate relative to the salt. Particularly preferably, the salt has about 2 equivalents of hydrate relative to the salt.

In a third aspect of the present invention, there is provided a method of preparing crystals of a sulfate salt of a compound of Chemical Formula 1, which comprises the steps of: mixing the compound of Chemical Formula 1 with a solvent selected from water, alcohols, ketones, or mixtures thereof, and adding a sulfuric acid solution to form a suspension; stirring the suspension and then separating it to obtain crystals.

The suspension may be formed by any method known in the art, and the present invention employs stirring and slurrying to form the suspension.

The stirring process for the formation of the suspension is usually carried out at 20-60 °C for 1-72 h. To ensure a more complete reaction, a two-stage stirring process can be used, e.g., stirring at 40-60 °C for 1-2 h followed by stirring at 20-30 °C for 1-24 h.

The salt of the compound of Chemical Formula 1 involved in the present invention is a polar substance, so the selection of a polar solvent can enable the salt to dispersed better in the system. Water, alcohols and ketones are all solvents with certain polarity, and any of these solvents and their mixtures can be selected as needed to achieve the appropriate polarity of the solvent to promote better dispersion of the salt in the system.

In a preferred embodiment, the solvent is a mixture of water and ketones.

In a preferred embodiment, the solvent is a mixture of water and acetone.

In the preferred embodiment, the volume ratio of water to acetone is 1/99 to 30/70.

In the preferred embodiment, the volume ratio of water to acetone is 5/95 to 10/90.

In preferred embodiments, the addition of the sulfuric acid solution is preferably 1.1 to 2.1 equivalents.

The sulfuric acid solution is usually selected as an aqueous solution of sulfuric acid or an organic solution of sulfuric acid, depending on the polarity of the solvent added to form the suspension.

Those skilled in the art recognize that there is an equilibrium between the sulfuric acid and the free base moiety in which protons may be present, depending on the conditions (e.g., solvent, temperature, etc.) and the strength of the acid. For example, under certain conditions, sulfuric acid donates one or more protons to the basic site of the free base to form weak interactions, so that the protons are shared between the acid and the free base. The addition of a slight excess amount of acid causes the sulfuric acid to react sufficiently with the free base moiety to fully convert the free base moiety into a salt. As the reaction proceeds, part of the sulfuric acid is consumed, leading to a decrease in its concentration and a reduction in reactivity, so an appropriate excess of sulfuric acid is required. However, the excessive use of sulfuric acid will bring more waste to the subsequent processing and increase the treatment cost. Therefore, it is preferable to add the sulfuric acid solution in the range of 1.1 to 2.1 equivalents.

The method of separating the crystals disclosed in the present invention may be any separation method known in the art, such as decantation, filtration, centrifugation, gravity sedimentation, etc., and a suitable separation method is generally selected according to the fluidity of the suspension and the particle size of the solid particles. The commonly used methods are filtration and centrifugation.

The solid obtained after separation was placed in a vacuum oven and dried at 30-60 °C for 0.5-24 hours.

In a fourth aspect of the present invention, there is provided a method of preparing the crystal of a sulfate salt of a compound of Chemical Formula 1, comprising: mixing the compound of Chemical Formula 1 with a solvent selected from water, alcohols, ketones, and mixtures thereof, and forming a suspension by adding a sulfuric acid solution; adding a sulfate salt polymorph A of the compound of Chemical Formula 1 as a seed crystal to the suspension; stirring the suspension and then isolating the crystals by separation.

In preferred embodiments, the solvent is selected from water, methanol, ethanol, n-propanol, isopropanol, 1-butanol, tert-butanol, acetone, butanone, 2-pentanone or mixtures thereof.

In preferred embodiments, the solvent is selected from water, ethanol, acetone or mixtures thereof.

In a fifth aspect of the present invention, there are provided pharmaceutical compositions comprising a salt as described in the first aspect or a crystal as described in the second aspect and a pharmaceutically acceptable excipient.

In the following examples, experimental methods without specified conditions are generally carried out under conventional conditions or according to the manufacturer's recommended procedures.

Unless otherwise indicated, the raw materials or reagents used in the examples were purchased from Sinopharm Chemical Reagent Co., Ltd.

Unless otherwise indicated, the reagents described are used directly without purification.

The following techniques are used to identify, characterize or analyze crystals: X-ray powder diffraction (XRPD), thermogravimetric analysis (TGA), differential scanning calorimetry (DSC), nuclear magnetic resonance (NMR) and chromatographic techniques such as HPLC.

The XRPD spectra were acquired on a Bruker D8 ADVANCE diffractometer, and the method parameters of the X-ray powder diffraction are as follows:
X-ray reflection parameters: Cu, Kα
Tube voltage: 40 kilovolts (kV)
Tube current: 40 milliamps (mA)
Slit: 2# scattering slit: 1°, 3# anti-scattering slit: 1°, 4# receiving slit: 0.3 mm
Scanning mode: step
Step angle:0.02°,
Sampling time:0.2 s/step
Scanning range: from 3.0 to 40.0°.
TGA Analytical Methods, Instrument Information:
   Model: TA DISCOVERY TGA 55
   Manufacturer: TA instruments
   parameter settings
   Sample Tray: Platinum HT
   Sample weight: 10 mg
   Heating rate: 10 ° C/min
   Temperature Scanning Range: RT-400 ° C
   Balance purge gas flow: 40 mL/min
   Furnace purge gas flow:60 mL/min
   DSC analytical methods, instrument information:
      Model: TA DISCOVERY DSC 250
      Manufacturer: TA instruments
      parameter settings
      Sample Tray: Tzero Aluminum
      Cover: Yes
      Sample weight: 3-5 mg
      Heating rate: 10 ° C/min
      Temperature scanning range:0-400 °C
      Nitrogen purge flow: 50 mL/min

### Preparation of compounds of Chemical Formula 1

The compound of Chemical Formula 1 can be prepared according to the method documented in WO2020155842A1, or according to the exemplary preparation method described below.

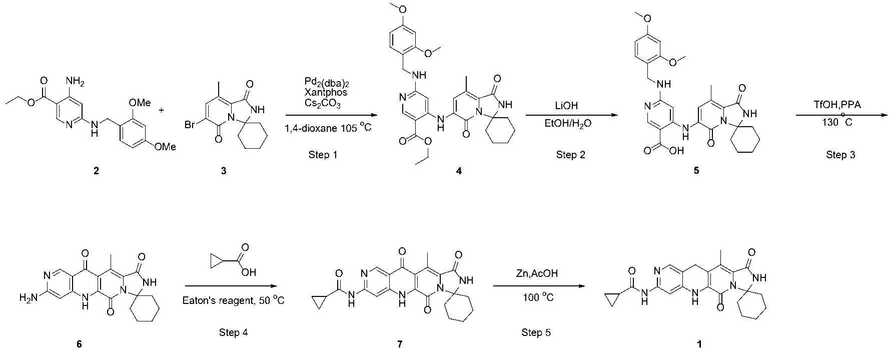

### Step 1: Synthesis of compound 4

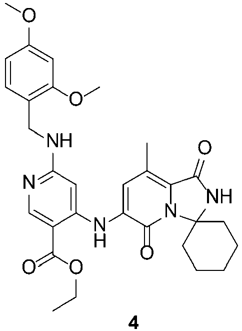

A three-necked flask was charged with Ethyl 4-amino-6-((2,4-dimethoxybenzyl)amino)nicotinate (Compound 2, 45.0 g, prepared according to the synthesis of Intermediate A as documented in WO2020155842A1), 6'-bromo-8'-methyl-2'H-spiro[cyclohexane-1,3'-imidazo[1,5-a]pyridine]-1', 5'-dione (Compound **3,** 136.8 g, prepared according to the method of synthesis of Intermediate B as documented in WO2020155842A1), Pd₂(dba)₃ (7.44 g), XantPhos (9.42 g) and cesium carbonate (265.5 g). The mixture was degassed and purged with nitrogen three times. 1,4-dioxane (2700 mL) was added and the reaction mixture was heated and refluxed for 22 hours under nitrogen. The reaction mixture was cooled to room temperature and poured into a mixture of water and ethyl acetate. The mixture was filtered after stirring for 0.5 h at room temperature. The filter cake was dried at 60 °C to give the desired compound **4** (204 g, purity 98.05%, yield 87.8%) as a gray solid.

LCMS: (ESI) [M+H]⁺ = 562.3.

### Step 2: Synthesis of compound 5

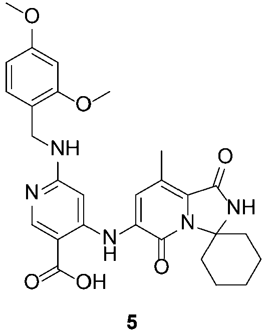

6-((2,4-dimethoxybenzyl)amino)-4-((8'-methyl-1',5'-dioxa-1',5'-dihydr o-2'H-spiro[cyclohexane-1,3'-Imidazo[1,5-a]pyridin]-6'-yl)amino) nicotinic acid ethyl ester (Compound 4, 203.3 g) was mixed in a mixture of ethanol and water (7.5 L), and lithium hydroxide (60 g) was added. The reaction mixture was heated and stirred for 2 hours at 80 °C. The reaction solution was cooled to room temperature and acidified to pH=3~4 by adding 6 M HCl dropwise. The mixture was stirred for half an hour and then filtered. The filter cake was washed with ethanol:water=1:1 and dried at 60 °C. The target compound **5** (219.3 g, 100% yield) was obtained as a yellow solid.

LCMS: (ESI) [M+H]⁺ = 534.3.

### Step 3: Synthesis of compound 6

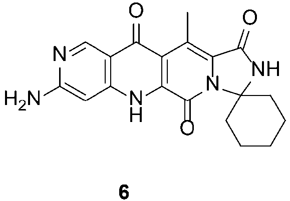

A mixed solution of polyphosphoric acid (174 g) and trifluoromethanesulfonic acid (1740 mL) was heated to 130 °C followed by adding 6-((2,4-dimethoxybenzyl)amino)-4-((8'-methyl-1',5'-dioxo-1',5'-dihydro-2'H-spiro[cyclohexane-1,3'-imidazo[1,5-a]pyridin]-6'-yl)aminonic otinic acid (Compound **5,** 200 g). The reaction mixture was heated and stirred at 130 °C for 0.5 h. After the mixture was cooled to 15 °C, the mixture was slowly added to ice-water, adjusted to pH=7 and filtered. The filter cake was washed with water and dried to give compound **6** (85 g, 62% yield) as a yellow solid.

LCMS: (ESI) [M+H]⁺ = 366.2.

¹H NMR: (400 MHz, DMSO-d6) δ 11.49 (s, 1H), 10.13 (s, 1H), 8.78 (s, 1H), 6.70 (s, 2H), 6.59 (s, 1H), 3.02-2.90 (m, 5H), 1.79-1.60 (m, 5H), 1.52-1.49 (m,2H), 1.28-1.20 (m, 1H).

### Step 4: Synthesis of compound 7

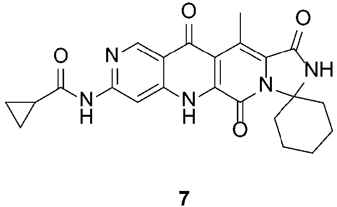

At room temperature, 8-amino-12-methyl-1H-spiro[cyclohexane-1,3-imidazo[1,5:1,6]pyrido[3,4-b][1,6]naphthyridine]-1,5,11'(2H,6H)-trione (Compound **6,** 75 g) was dissolved in Eaton's reagent (1125 mL) and cyclopropane carboxylic acid (35.4 g) was added to the reaction mixture. The reaction mixture was heated and stirred at 50 °C for 1 hour. The mixture was cooled to room temperature and slowly poured into ice-water (58 times volume). The mixture was stirred for 30 min and filtered. The solid obtained from filtration was dried at 60 °C for 16 h to yield the crude product of target compound 7 (98.7 g, purity 95.95%) as a yellow solid.

LCMS: (ESI) [M+H]⁺ = 434.2.

¹H NMR: (400 MHz, DMSO-d6) δ 12.09 (s, 1H), 11.13 (s, 1H), 10.21 (s, 1H), 9.03 (s, 1H), 8.54 (s, 1H), 3.02-2.90 (m, 5H), 2.02-2.36 (m, 1H), 1.80-1.59 (m,5H), 1.52-1.49 (m,2H), 1.30-1.22 (m, 1H), 0.85-0.87 (m, 4H).

### Step 5: Synthesis of compound 1 of Chemical Formula 1

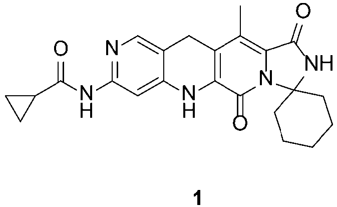

N-(12-methyl-1,5,11-trioxo-2,5,6,11-tetrahydro-1H-spiro[cyclohexane -1,3-imidazo[1,5:1,6]pyrido[3,4-b][1,6]naphthopyridin]-8-yl)cyclopropane carboxamide (Compound **7,** 70.0 g) was dissolved in acetic acid (1600 mL) and Zinc powder (106 g) was added to the reaction mixture at 25 °C. The reaction mixture was heated and stirred at 100 °C for 2 hours. The reaction mixture was filtered, the filter cake was washed with acetic acid and dried at room temperature to yield 100 g of the crude product of compound **1.**

LCMS: (ESI) [M+H]⁺ = 440.2.

¹H NMR: (400 MHz, DMSO-d6) δ 10.45 (s, 1H), 10.01 (s, 1H), 9.01 (s, 1H), 7.90 (s, 1H), 7.78 (s, 1H), 3.96 (s, 2H), 3.10-2.93 (m, 2H), 2.42 (s, 3H), 2.02-1.92 (m, 1H), 1.79-1.56 (m,5H), 1.47-1.39 (m,2H), 1.280-1.23 (m, 1H), 0.82-0.77 (m, 4H).

### Comparative Example 1 Crystallization preparation of Compound 1

70 g of the crude product of compound **1** obtained by the above experimental method was taken, and dichloromethane (560 mL) was added at room temperature to fully dissolve it. The insoluble matter was removed by filtration. The solution was cooled to 0 °C to precipitate solids, followed by filtration and vacuum drying to obtain 38.2 g of compound **1** crystals. The resulting crystals were sampled for analysis.

The X-ray powder diffraction spectra of the obtained solid products were examined as shown in Fig. 1, and the X-ray powder diffraction patterns of the obtained crystals exhibited characteristic peaks at diffraction angles (2θ±0.20°) of 6.225°, 9.536°, 11.212°, 14.415°, 15.303°, 15.779°, 16.452°, 19.290°, 20.915°, 22.133°, 23.295°, 24.532°, 25.473°, 26.705°, 27.776°, 28.871°, 29.596°, and 30.915°, 22.133°, 23.295°, 24.532°, 25.473°, 26.705°, 27.776°, 28.871°, 29.596°, and 30.353 °. This crystal form is defined as the polymorph I of compound **1.**

Example 1 Preparation of polymorph A of sulfate salt of Compound **1**

In a 1 L round-bottomed flask, 20 g of compound polymorph I obtained in Comparative Example 1 and 300 mL of acetone-water mixture, acetone/water (v/v) = 95/5, were added dropwise with 1.1 eq. of acetone sulfate (1.1 eq. of concentrated sulfuric acid diluted 10-fold with acetone) under slow stirring conditions to form a suspension. The suspension was stirred at 50 °C for 1 h and then stirred at 25 °C for 18 h. The suspension was filtered and the filter cake was vacuum dried at 50 °C for 18 h. 24.4 g of yellow sulfate crystals were obtained. According to the results of ion chromatographic analysis, the molar ratio of compound **1**/sulfate in the crystals was 1/1.0.

The resulting sulfate crystals of compound **1** were characterized by XRPD, and the X-ray powder diffractograms are shown in Figure 2, with characteristic peaks at diffraction angles (2θ±0.20°) of 7.439°, 10.519°, 12.329°, 13.503°, 14.825°, 15.723°, 17.430°, 18.334°, 19.700°, 20.048 °, 21.302°, 24.099°, 24.567°, 25.551°, 27.096°, 27.933°, and 29.770°; or further having characteristic peaks at diffraction angles (2θ ± 0.20°) of 11.231°, 15.342°, 18.812°, 22.307°, 23.194°, 25.053 °, 26.040°, 30.331°, 30.985°, 32.097°, 32.754°, 33.690°, 34.834°, and 35.833° with characteristic peaks. This crystal is defined as polymorph A of sulfate salt. The analyzed data of XRPD pattern of polymorph A of sulfate salt of compound 1 is shown in Table 1 below.

**Table 1**

| No | Diffraction angle (2θ) [°] | Relative strength (%) | Intensity |
|---|---|---|---|
| 1 | 7.439° | 100.0% | 1135.38 |
| 2 | 10.519° | 28.3% | 381.58 |
| 3 | 11.231° | 6.0% | 148.72 |
| 4 | 12.329° | 46.4% | 558.353 |
| 5 | 13.503° | 39.0% | 482.746 |
| 6 | 14.825° | 46.9% | 571.987 |
| 7 | 15.342° | 20.5% | 301.43 |
| 8 | 15.723° | 88.7% | 1006.55 |
| 9 | 17.430° | 27.4% | 370.733 |
| 10 | 18.334° | 47.9% | 590.507 |
| 11 | 18.812° | 14.4% | 249.176 |
| 12 | 19.700° | 44.9% | 570.112 |
| 13 | 20.048° | 73.8% | 869.614 |
| 14 | 21.302° | 45.3% | 581.948 |
| 15 | 22.307° | 27.8% | 406.22 |
| 16 | 23.194° | 19.4% | 317.69 |
| 17 | 24.099° | 70.5% | 844.883 |
| 18 | 24.567° | 58.6% | 722.646 |
| 19 | 25.053° | 24.4% | 368.131 |
| 20 | 25.551° | 56.4% | 695.516 |
| 21 | 26.040° | 11.8% | 229.009 |
| 22 | 27.096° | 68.4% | 805.513 |
| 23 | 27.933° | 28.6% | 387.382 |
| 24 | 29.770° | 38.9% | 500.306 |
| 25 | 30.331° | 14.4% | 249.575 |
| 26 | 30.985° | 6.0% | 162.733 |
| 27 | 32.097° | 5.2% | 145.946 |
| 28 | 32.754° | 25.4% | 354.215 |
| 29 | 33.690° | 9.8% | 191.665 |
| 30 | 34.834° | 12.5% | 208.574 |
| 31 | 35.833° | 5.5% | 127.069 |

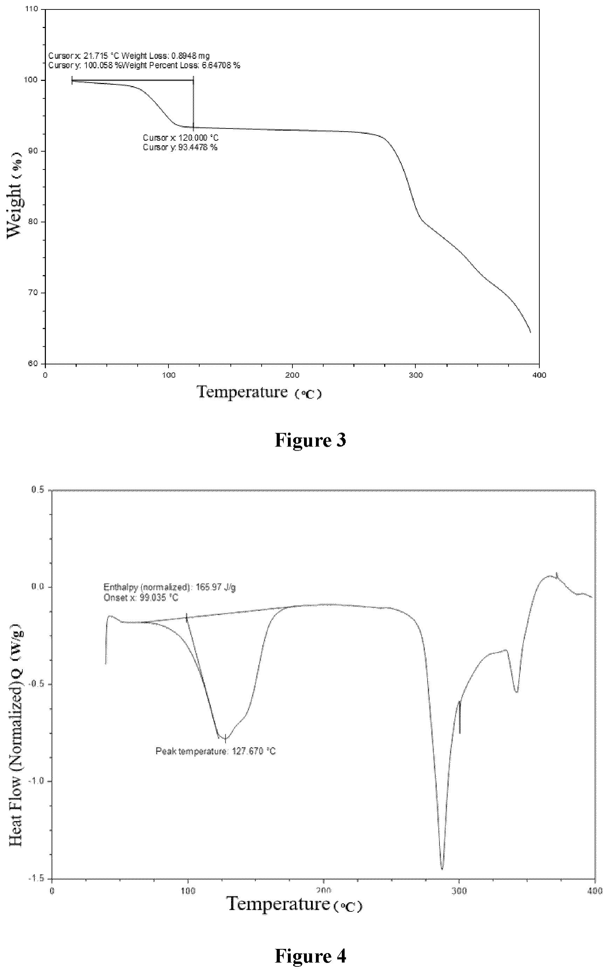

The TGA pattern of the obtained polymorph A is shown in Fig. 3. It can be observed that polymorph A of sulfate salt desolvates 6.6% of crystal water at 120 °C, and it can be calculated that the sulfate polymorph A contains two molecules of crystal water. The crystal is the dihydrate of sulfate polymorph A of compound **1.**

The DSC pattern of the obtained polymorph A is shown in Fig. 4. It can be observed that the sulfate polymorph A starts to melt at 99 °C, with a melting temperature of 127.67 °C and a heat of fusion of 165.97 J/g.

### Example 2 Preparation of the polymorph B of hydrochloride salt of Compound 1

In a 40 mL vial, 300 mg of polymorph I of compound obtained in Comparative Example 1 and 7 mL of acetonitrile were added, then 1.1 equivalents of hydrochloric acid acetonitrile solution (1.1 eq of concentrated hydrochloric acid diluted 10-fold with acetonitrile) was added dropwise to form a suspension under slow stirring conditions. The suspension was stirred at 25 °C for 72 h. The suspension was separated by centrifugation, and the lower solid layer after centrifugation was vacuum dried at 50 °C for 0.5 h. 330 mg of yellow hydrochloride crystals were obtained. According to the results of ion chromatography analysis, the compound **1**/hydrochloric acid (molar ratio) in the crystals was 1/1.1.

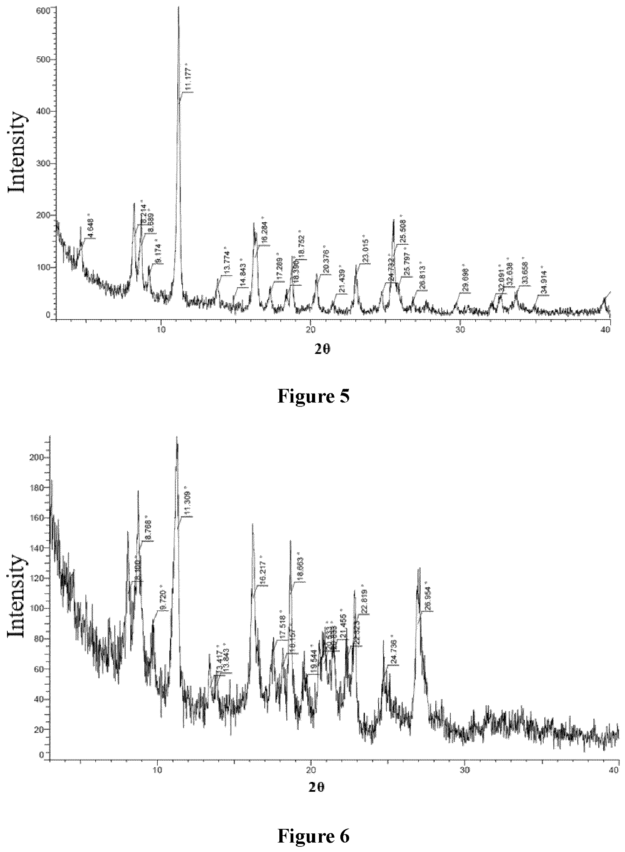

The resulting hydrochloride crystals of compound **1** were subjected to XRPD characterization and the X-ray powder diffractograms are shown in Fig. 5, with characteristic peaks at diffraction angles (2θ ± 0.20°) of 4.648°, 8.214°, 8.689°, 9.174°, 11.177°, 13.774°, 16.284°, 17.289°, 18.752°, 20.376°, 23.015°, 24.732°, 25.508°, 25.797°, 26.813°, 29.698°, 32.638°, and 33.658°. This crystal is defined as hydrochloride polymorph B. The analyzed data of the XRPD pattern of hydrochloride polymorph B of compound 1 is shown in Table 2 below.

**Table 2**

| No | Diffraction angle (2θ) [°] | Relative strength (%) | Intensity |
|---|---|---|---|
| 1 | 4.648° | 9.10% | 132.211 |
| 2 | 8.214° | 29.70% | 159.049 |
| 3 | 8.689° | 25.50% | 141.983 |
| 4 | 9.174° | 10.60% | 83.0324 |
| 5 | 11.177° | 100.00% | 413.726 |
| 6 | 13.774° | 9.90% | 60.9666 |
| 7 | 16.284° | 25.90% | 118.785 |
| 8 | 17.289° | 7.80% | 49.2168 |
| 9 | 18.752° | 19.70% | 91.9114 |
| 10 | 20.376° | 12.20% | 63.0724 |
| 11 | 23.015° | 18.30% | 83.5875 |
| 12 | 24.732° | 7.60% | 47.0445 |
| 13 | 25.508° | 26.80% | 121.686 |
| 14 | 25.797° | 10.20% | 58.9153 |
| 15 | 26.813° | 3.40% | 32.1715 |
| 16 | 29.698° | 4.00% | 27.8134 |
| 17 | 32.638° | 6.30% | 39.4223 |
| 18 | 33.658° | 6.40% | 41.0768 |

### Example 3 Preparation of the methanesulfonate polymorph B of Compound 1

In a 40 mL vial, 500 mg of compound polymorph I obtained in Comparative Example 1 and 10 mL of acetonitrile were added, then under slow stirring conditions, 1.1 eq. of acetone solution of methanesulfonic acid was added dropwise (1.1 eq. of methanesulfonic acid was taken and diluted 10-fold with acetone) to form a suspension. The suspension was stirred at 50 °C for 1 h and then stirred at 25 °C for 8 h. The suspension was filtered and the filter cake was vacuum dried at 50 °C for 2 h. 450 mg of yellow methanesulfonate crystals were obtained. According to the H-NMR analysis, the molar ratio of compound **1**/methanesulfonic acid in the crystals was 1/1.1.

The resulting methanesulfonate crystals of compound **1** were characterized by XRPD, and the X-ray powder diffractograms are shown in Fig. 6, with characteristic peaks at diffraction angles (2θ±0.20°) of 8.100°, 8.768°, 9.720°, 11.309°, 13.417°, 13.843°, 16.217°, 17.518°, 18.157°, 18.663°, 19.544°, 20.533°, 20.833°, 21.455°, 22.323°, 22.819°, 24.736° and 26.954°. This crystal is defined as polymorph B of mesylate salt. The analyzed data of XRPD pattern of polymorph B of mesylate salt of compound **1** is shown in Table 3 below.

**Table 3**

| No | Diffraction angle (2θ) [°] | Relative strength (%) | Intensity |
|---|---|---|---|
| 1 | 8.100° | 42.70% | 109.748 |
| 2 | 8.768° | 69.20% | 136.424 |
| 3 | 9.720° | 36.00% | 90.1331 |
| 4 | 11.309° | 100.00% | 152.613 |
| 5 | 13.417° | 13.30% | 47.6365 |
| 6 | 13.843° | 14.20% | 48.9182 |
| 7 | 16.217° | 62.30% | 106.626 |
| 8 | 17.518° | 30.90% | 70.6921 |
| 9 | 18.157° | 24.70% | 61.8553 |
| 10 | 18.663° | 67.40% | 109.22 |
| 11 | 19.544° | 15.80% | 48.129 |
| 12 | 20.533° | 27.80% | 63.3956 |
| 13 | 20.833° | 28.10% | 63.6625 |
| 14 | 21.455° | 35.60% | 71.3789 |
| 15 | 22.323° | 34.60% | 66.7938 |
| 16 | 22.819° | 55.10% | 87.6681 |
| 17 | 24.736° | 27.80% | 54.4802 |
| 18 | 26.954° | 56.00% | 89.7289 |

### Example 4 Preparation of polymorph A of formate salt of Compound 1

In a 5 mL vial, 100 mg of polymorph I of compound obtained in Comparative Example 1 and 0.1 mL of formic acid were added, slowly stirred until compound **1** was completely dissolved. The solution was filtered, 0.5 mL of deionized water was slowly added to the filtrate until sufficient solid was precipitated, and then filtration was carried out, and the filter cake was vacuum dried for 2 h at 30 °C to yield 80 mg of yellow formate crystals. According to the analysis of H-NMR results, the molar ratio of compound **1**/formic acid in the crystals was 1/1.0.

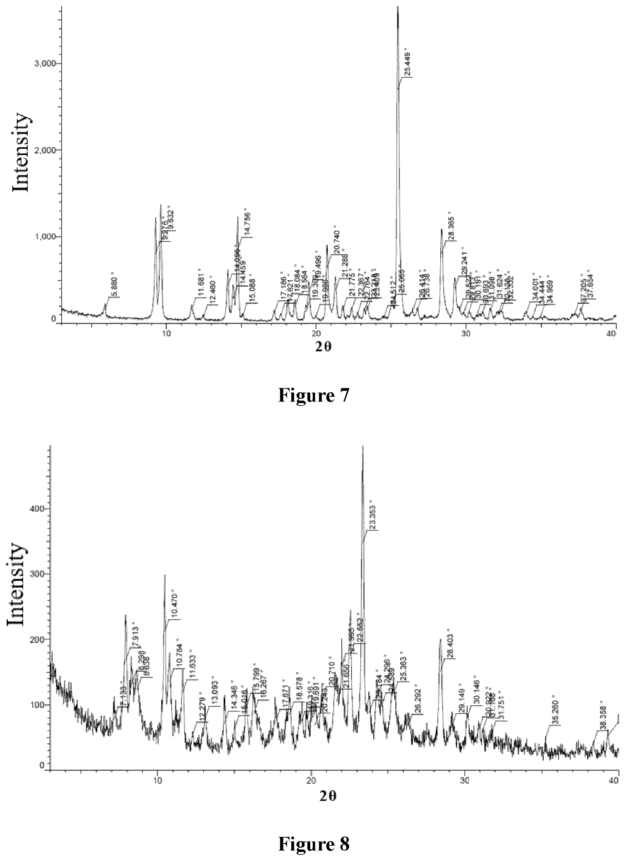

After XRPD characterization of the resulting formate crystals of compound **1**, the X-ray powder diffractograms are shown in Fig. 7, with characteristic peaks at diffraction angles (2θ±0.20°) of 5.880°, 9.276°, 9.632°, 11.681°, 12.460°, 14.096°, 14.459°, 14.756°, 15.088°, 17.186° , 17.621°, 18.084°, 18.564°, 19.496°, 19.986°, 20.740°, 21.288°, 21.775°, 22.367°, 22.764°, 23.409°, 24.512°, 25.065°, 25.449°, 26.414°, 26.738°, 28.365°, 29.241°, 29.813°, 30.191°, 30.693°, 31.098°, 31.624°, 32.332°, 34.001°, 34.444°, 34.969°, 37.205°, and 37.654°. This crystal is defined as polymorph A of formate. The analyzed data of the XRPD pattern of polymorph A of formate salt of compound **1** is shown in Table 4 below.

**Table 4**

| No | Diffraction angle (2θ) [°] | Relative strength (%) | Intensity |
|---|---|---|---|
| 1 | 5.880° | 3.00% | 159.883 |
| 2 | 9.276° | 28.40% | 804.775 |
| 3 | 9.632° | 33.20% | 929.869 |
| 4 | 11.681° | 4.30% | 155.798 |
| 5 | 12.460° | 1.00% | 67.9703 |
| 6 | 14.096° | 14.80% | 431.917 |
| 7 | 14.459° | 9.50% | 292.474 |
| 8 | 14.756° | 30.70% | 851.933 |
| 9 | 15.088° | 1.90% | 89.8323 |
| 10 | 17.186° | 3.10% | 116.675 |
| 11 | 17.621° | 1.90% | 90.4037 |
| 12 | 18.084° | 6.00% | 202.342 |
| 13 | 18.564° | 5.40% | 186.791 |
| 14 | 19.496° | 12.00% | 359.531 |
| 15 | 19.986° | 1.40% | 79.1715 |
| 16 | 20.740° | 22.80% | 649.156 |
| 17 | 21.288° | 12.30% | 379.097 |
| 18 | 21.775° | 3.80% | 155.707 |
| 19 | 22.367° | 3.70% | 154.179 |
| 20 | 22.764° | 2.30% | 115.36 |
| 21 | 23.409° | 3.80% | 153.986 |
| 22 | 24.512° | 0.50% | 64.5325 |
| 23 | 25.065° | 5.30% | 197.581 |
| 24 | 25.449° | 100.00% | 2699.33 |
| 25 | 26.414° | 3.10% | 140.746 |
| 26 | 26.738° | 3.50% | 149.083 |
| 27 | 28.365° | 27.90% | 807.305 |
| 28 | 29.241° | 12.70% | 403.769 |
| 29 | 29.813° | 1.80% | 108.459 |
| 30 | 30.191° | 2.90% | 131.482 |
| 31 | 30.693° | 1.10% | 76.985 |
| 32 | 31.098° | 2.60% | 119.216 |
| 33 | 31.624° | 3.50% | 145.497 |
| 34 | 32.332° | 2.90% | 125.871 |
| 35 | 34.001° | 2.30% | 107.07 |
| 36 | 34.444° | 0.70% | 68.6332 |
| 37 | 34.969° | 1.00% | 76.06 |
| 38 | 37.205° | 1.50% | 89.0472 |
| 39 | 37.654° | 3.50% | 141.65 |

### Example 5 Preparation of polymorph A of sulfate salt of Compound 1

In a 500 mL round-bottomed flask, 5 g of polymorph I of compound obtained in Comparative Example 1 and 100 mL of acetone-water mixture with acetone/water (v/v) = 95/5 were added, and then 1.1 equivalents of sulfuric acid acetone solution were added dropwise with slow stirring (1.1 eq of concentrated sulfuric acid was diluted 10-fold with acetone). To the suspension, 50 mg of sulfate polymorph A was added as the seed crystal, and the suspension was stirred at 50°C for 1 h and then transferred to 25°C for 24 h. The suspension was filtered, and the filter cake was vacuum dried at 50 °C for 18 h. 6.1 g of yellow sulfate crystals with high crystallinity were obtained. According to the results of ion chromatographic analysis, the compound **1**/sulfate (molar ratio) in the crystals was 1/1.0.

The resulting sulfate crystals of compound **1** were characterized by XRPD and determined to be the polymorph A of sulfate salt.

The crystals were analyzed as dihydrate of polymorph A of sulfate salt of compound **1.**

### Example 6 Preparation of sulfate polymorph A of Compound 1

In a 5 mL vial, 100 mg polymorph I of compound obtained in Comparative Example 1 and 1 mL of ethanol were added, and then 1.1 equivalents of ethanol solution of sulfuric acid (1.1 eq of concentrated sulfuric acid was diluted 10-fold with ethanol) was added dropwise under slow stirring conditions to form a suspension. The suspension was stirred at 25°C for 48 h. The suspension was centrifuged and the lower solid layer was dried under vacuum at 50°C for 2 h. 110 mg of yellow sulfate crystals were obtained. According to the results of ion chromatographic analysis, the molar ratio of compound **1**/sulfate in the crystals was 1/1.1.

The resulting sulfate crystals of compound **1** were characterized by XRPD and determined to be the polymorph A of sulfate.

The crystals were analyzed as dihydrate of polymorph A of sulfate salt of compound **1.**

### Example 7 Preparation of sulfate polymorph B of Compound 1

In a 5 mL vial, 100 mg of compound polymorph I obtained in Comparative Example 1 and 1 mL of acetonitrile were added, and then 1.1 eq of acetonitrile sulfate solution (1.1 eq of concentrated sulfuric acid diluted 10-fold with acetonitrile) was added dropwise to form a suspension under slow stirring conditions. The suspension was stirred at 25 °C for 48 h. The suspension was separated by centrifugation, and the lower solid layer after centrifugation was vacuum dried at 50 °C for 2 h. 103 mg of yellow sulfate crystals were obtained. According to the results of ion chromatographic analysis, the molar ratio of compound **1**/sulfate in the crystals was 1/0.7.

The resulting sulfate crystals of compound **1** were characterized by XRPD and the X-ray powder diffractograms are shown in Fig. 8, with characteristic peaks at diffraction angles (2θ ± 0.20°) of 7.133°, 7.913°, 8.298°, 8.638°, 10.471°, 10.784°, 11.633°, 12.279°, 13.093°, 14.346°, 15.016°, 15.799°, 16.267°, 17.671°, 18.578°, 19.316°, 19.691°, 20.710°, 21.656°, 21.995°, 22.552°, 23.353°, 23.784°, 24.296°, 24.589°, 25.363°, 26.292°, 28.403°, 29.149°, 30.146°, 30.922°, 31.751°, 35.260°, 38.358°, and 39.295°. This crystal is defined as polymorph B of sulfate salt. The resolved data of the XRPD pattern of polymorph B of sulfate salt of compound **1** is shown in Table 5 below.

**Table 5**

| No | Diffraction angle (2θ) [°] | Relative strength (%) | Intensity |
|---|---|---|---|
| 1 | 7.133° | 6.0% | 77.0516 |
| 2 | 7.913° | 37.5% | 168.035 |
| 3 | 8.298° | 25.1% | 131.805 |
| 4 | 8.638° | 22.4% | 122.678 |
| 5 | 10.471° | 55.9% | 211.488 |
| 6 | 10.784° | 30.2% | 135.921 |
| 7 | 11.633° | 26.7% | 118.671 |
| 8 | 12.279° | 5.9% | 53.6531 |
| 9 | 13.093° | 15.0% | 78.3981 |
| 10 | 14.346° | 12.7% | 71.1911 |
| 11 | 15.016° | 9.5% | 64.4132 |
| 12 | 15.799° | 20.3% | 98.4852 |
| 13 | 16.267° | 16.5% | 87.8751 |
| 14 | 17.671° | 11.5% | 73.5157 |
| 15 | 18.578° | 16.0% | 84.6279 |
| 16 | 19.316° | 10.5% | 70.9032 |
| 17 | 19.691° | 11.7% | 77.2702 |
| 18 | 20.710° | 20.8% | 108.182 |
| 19 | 21.656° | 17.7% | 104.495 |
| 20 | 21.995° | 36.0% | 159.55 |
| 21 | 22.552° | 40.4% | 174.913 |
| 22 | 23.353° | 100.0% | 347.814 |
| 23 | 23.784° | 9.6% | 88.0845 |
| 24 | 24.296° | 18.2% | 111.576 |
| 25 | 24.589° | 14.3% | 98.8319 |
| 26 | 25.363° | 21.3% | 115.775 |
| 27 | 26.292° | 5.9% | 66.3905 |
| 28 | 28.403° | 37.4% | 150.626 |
| 29 | 29.149° | 9.1% | 66.7293 |
| 30 | 30.146° | 14.4% | 79.5253 |
| 31 | 30.922° | 7.9% | 61.9227 |
| 32 | 31.751° | 6.4% | 55.8031 |
| 33 | 35.260° | 8.6% | 48.9906 |
| 34 | 38.358° | 5.3% | 40.2915 |
| 35 | 39.295° | 9.6% | 52.3793 |

The crystals were analyzed as acetonitrile solvent compounds of polymorph B of sulfate salt of compound **1.**

### Example 8 Preparation of sulfate polymorph C of Compound 1

500 mg of sulfate polymorph A prepared according to Example 1 and 3 mL of acetonitrile were added to a 10 mL vial, suspended and pulped for 3 days at 25 °C. The suspension was filtered, and the filter cake was vacuum dried at 40 °C for 2 hours to obtain 278 mg of yellow sulfate crystals. According to the results of ion chromatographic analysis, the molar ratio of compound **1**/sulfate in the crystals was 1/1.1.

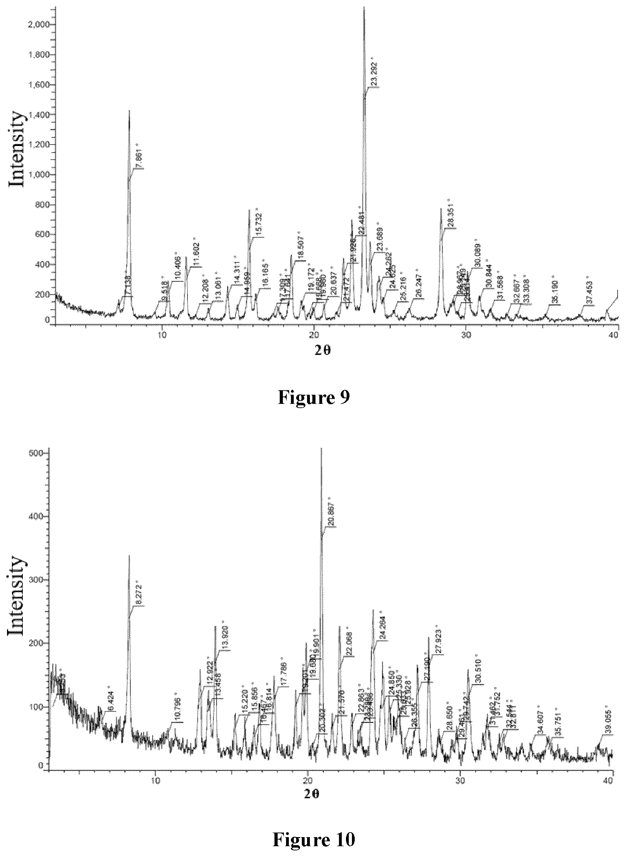

The resulting sulfate crystals of compound **1** were characterized by XRPD, and the X-ray powder diffractograms are shown in Fig. 9, with characteristic peaks at diffraction angles (2θ±0.20°) of 7.138°, 7.861°, 10.406°, 11.602°, 13.061°, 14.311°, 14.959°, 15.732°, 16.165°, 17.641° , 18.507°, 19.172°, 19.960°, 20.637°, 21.926°, 22.481°, 23.292°, 23.689°, 24.262°, 24.525°, 25.216°, 26.247°, 28.351°, 29.149°, 30.089°, 30.844° and 31.568°. This crystal is defined as polymorph C of sulfate salt. The analyzed data of the XRPD pattern of polymorph C of sulfate salt of compound **1** is shown in Table 6 below.

**Table 6**

| No | Diffraction angle (2θ) [°] | Relative strength (%) | Intensity |
|---|---|---|---|
| 1 | 7.138° | 3.4% | 106.286 |
| 2 | 7.861° | 62.7% | 956.18 |
| 3 | 10.406° | 11.1% | 206.204 |
| 4 | 11.602° | 17.9% | 301.076 |
| 5 | 13.061° | 2.6% | 74.1207 |
| 6 | 14.311° | 9.8% | 179.466 |
| 7 | 14.959° | 4.4% | 104.591 |
| 8 | 15.732° | 31.8% | 498.911 |
| 9 | 16.165° | 8.4% | 157.64 |
| 10 | 17.641° | 3.6% | 90.2056 |
| 11 | 18.507° | 20.8% | 342.287 |
| 12 | 19.172° | 6.0% | 130.608 |
| 13 | 19.960° | 3.3% | 86.7927 |
| 14 | 20.637° | 4.9% | 106.898 |
| 15 | 21.926° | 19.0% | 325.802 |
| 16 | 22.481° | 31.5% | 510.514 |
| 17 | 23.292° | 100.0% | 1501.38 |
| 18 | 23.689° | 23.6% | 399.903 |
| 19 | 24.262° | 12.4% | 234.196 |
| 20 | 24.525° | 6.8% | 150.953 |
| 21 | 25.216° | 1.9% | 70.3403 |
| 22 | 26.247° | 3.7% | 90.6072 |
| 23 | 28.351° | 35.7% | 559.156 |
| 24 | 29.149° | 5.5% | 124.669 |
| 25 | 30.089° | 17.1% | 292.03 |
| 26 | 30.844° | 7.6% | 156.015 |
| 27 | 31.568° | 2.6% | 79.8942 |

The crystals were analyzed as acetonitrile solvent compounds of polymorph C of sulfate salt of compound **1.**

### Example 9 Preparation of sulfate polymorph D of compound 1

In a 5 mL vial, 50 mg of sulfate polymorph A prepared according to Example 1 and 1 mL of DMF were added, and the mixture was suspended and pulped for 3 weeks at 25 °C. The suspension was filtered, and the filter cake was vacuum dried at 40 °C for 2 hours to obtain 45 mg of yellow sulfate crystals. According to the results of ion chromatographic analysis, the molar ratio of compound **1**/sulfate in the crystals was 1/1.0.

The resulting sulfate crystals of compound **1** were characterized by XRPD, and the X-ray powder diffractograms are shown in Fig. 10, with characteristic peaks at diffraction angles (2θ±0.20°) of 6.424°, 8.272°, 10.796°, 12.922°, 13.458°, 13.920°, 15.220°, 15.856°, 16.467°, 16.814 °, 17.786°, 19.201°, 19.680°, 19.901°, 20.302°, 20.867°, 21.570°, 22.068°, 22.863°, 23.436°, 24.264°, 24.850°, 25.330°, 25.633°, 26.355°, 27.190°, 27.923°, 28.650°, 29.742°, 30.510°, 31.462°, 31.752°, 32.544°, 32.811°, 34.607°, 35.751°, and 39.055°. This crystal is defined as polymorph D of sulfate salt. The resolved data of the XRPD pattern of polymorph D of sulfate salt of compound 1 is shown in Table 7 below.

**Table 7**

| No | Diffraction angle (2θ) [°] | Relative strength (%) | Intensity |
|---|---|---|---|
| 1 | 6.424° | 6.9% | 73.1022 |
| 2 | 8.272° | 55.5% | 239.273 |
| 3 | 10.796° | 5.8% | 55.7697 |
| 4 | 12.922° | 23.9% | 112.82 |
| 5 | 13.458° | 18.4% | 93.3394 |
| 6 | 13.920° | 41.2% | 168.87 |
| 7 | 15.220° | 12.0% | 67.5887 |
| 8 | 15.856° | 13.6% | 71.6602 |
| 9 | 16.467° | 8.5% | 52.4244 |
| 10 | 16.814° | 14.1% | 70.6516 |
| 11 | 17.786° | 26.4% | 111.361 |
| 12 | 19.201° | 22.8% | 100.918 |
| 13 | 19.680° | 29.4% | 123.756 |
| 14 | 19.901° | 38.8% | 155.754 |
| 15 | 20.302° | 4.4% | 38.1622 |
| 16 | 20.867° | 100.0% | 363.349 |
| 17 | 21.570° | 12.6% | 66.8003 |
| 18 | 22.068° | 40.1% | 159.337 |
| 19 | 22.863° | 13.9% | 69.4895 |
| 20 | 23.436° | 11.1% | 63.0555 |
| 21 | 24.264° | 45.7% | 184.423 |
| 22 | 24.850° | 19.7% | 97.338 |
| 23 | 25.330° | 17.9% | 91.4389 |
| 24 | 25.633° | 10.5% | 65.925 |
| 25 | 26.355° | 5.7% | 46.6785 |
| 26 | 27.190° | 27.7% | 119.325 |
| 27 | 27.923° | 41.2% | 162.414 |
| 28 | 28.650° | 6.4% | 41.6996 |
| 29 | 29.742° | 11.9% | 57.0073 |
| 30 | 30.510° | 28.9% | 115.824 |
| 31 | 31.462° | 8.5% | 49.2253 |
| 32 | 31.752° | 13.0% | 64.2235 |
| 33 | 32.544° | 7.4% | 44.6884 |
| 34 | 32.811° | 7.2% | 44.3045 |
| 35 | 34.607° | 5.0% | 35.3769 |
| 36 | 35.751° | 3.7% | 32.1592 |
| 37 | 39.055° | 4.5% | 36.0327 |

The crystals were analyzed as DMF solvent compounds of polymorph D of sulfate salt of compound **1.**

### Example 10 Preparation of sulfate polymorph E of Compound 1

In a 10 mL vial, 10 mg of sulfate polymorph A prepared according to Example 1 and 0.8 mL of DMSO were added, slowly stirred until completely dissolved. The solution was filtered, and 6 volumes of dichloromethane was slowly added to the filtrate until a sufficient amount of solid was precipitated. Then filtration was carried out, and the filter cake was vacuum dried for 2 hours at 40 °C to obtain 8 mg of yellow sulfate crystals. According to the results of ion chromatographic analysis, the molar ratio of compound **1**/sulfate in the crystals was 1/1.0.

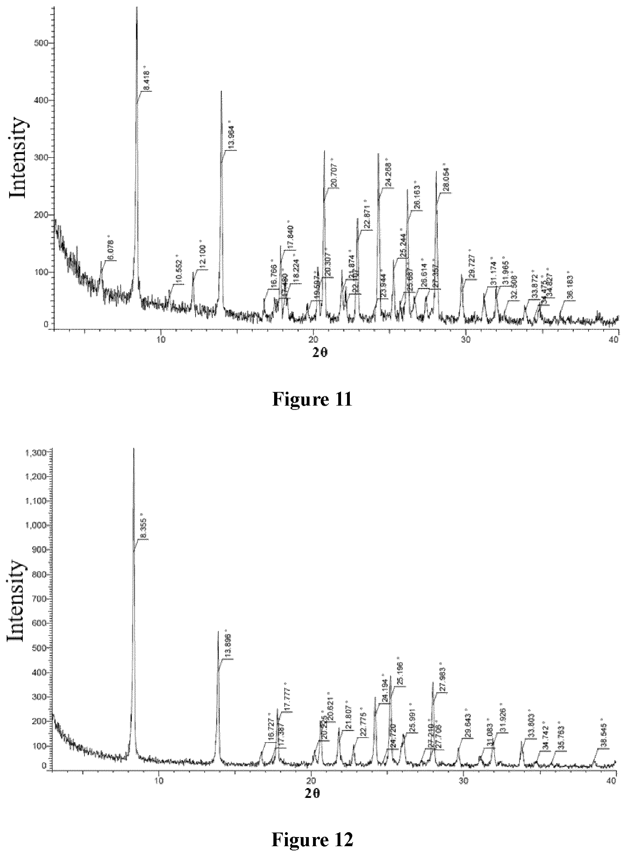

The resulting sulfate crystals of compound **1** were characterized by XRPD, and the X-ray powder diffractograms are shown in Fig. 11, with characteristic peaks at diffraction angles (2θ±0.20°) of 6.078°, 8.418°, 10.552°, 12.100°, 13.964°, 16.766°, 17.480°, 17.840°, 18.224°, 19.597 °, 20.307°, 20.707°, 21.874°, 22.107°, 22.871°, 23.944°, 24.268°, 25.244°, 25.687°, 26.163°, 26.614°, 27.357°, 28.054°, 29.727°, 31.174°, 31.965°, 32.508°, 33.872°, 34.827° and 36.183°. This crystal is defined as polymorph E of sulfate salt. The resolved data of the XRPD pattern of polymorph E of sulfate salt of compound **1** is shown in Table 8 below.

**Table 8**

| No | Diffraction angle (2θ) [°] | Relative strength (%) | Intensity |
|---|---|---|---|
| 1 | 6.078° | 9.3% | 97.7967 |
| 2 | 8.418° | 100.0% | 393.314 |
| 3 | 10.552° | 6.0% | 56.3094 |
| 4 | 12.100° | 14.8% | 82.0921 |
| 5 | 13.964° | 77.2% | 290.316 |
| 6 | 16.766° | 9.1% | 47.9035 |
| 7 | 17.480° | 4.1% | 31.7781 |
| 8 | 17.840° | 28.5% | 115.824 |
| 9 | 18.224° | 11.5% | 57.6291 |
| 10 | 19.597° | 3.2% | 28.0409 |
| 11 | 20.307° | 14.7% | 68.4453 |
| 12 | 20.707° | 60.3% | 224.334 |
| 13 | 21.874° | 14.0% | 64.7584 |
| 14 | 22.107° | 6.8% | 39.7088 |
| 15 | 22.871° | 39.6% | 151.705 |
| 16 | 23.944° | 3.9% | 31.1804 |
| 17 | 24.268° | 60.0% | 224.754 |
| 18 | 25.244° | 23.5% | 102.182 |
| 19 | 25.687° | 6.1% | 42.5854 |
| 20 | 26.163° | 47.9% | 185.346 |
| 21 | 26.614° | 8.5% | 49.6582 |
| 22 | 27.357° | 8.7% | 48.7643 |
| 23 | 28.054° | 58.0% | 216.247 |
| 24 | 29.727° | 15.0% | 65.5248 |
| 25 | 31.174° | 11.0% | 52.7194 |
| 26 | 31.965° | 11.1% | 53.4958 |
| 27 | 32.508° | 4.0% | 28.1451 |
| 28 | 33.872° | 5.6% | 30.2056 |
| 29 | 34.827° | 6.5% | 33.0003 |
| 30 | 36.183° | 5.2% | 28.231 |

The crystals were analyzed as DMSO solvates of polymorph E of sulfate salt of compound **1.**

### Example 11 Preparation of polymorph F of sulfate salt of Compound 1

In a 10 mL vial, 10 mg of sulfate polymorph A prepared according to Example 1 and 0.8 mL of DMSO were added, slowly stirred until completely dissolved. The solution was filtered, and 6 volumes of anisole was slowly added to the filtrate until a sufficient amount of solids was precipitated. Then filtration was carried out, and the filter cake was vacuum dried for 2 hours at 40 °C to obtain 9 mg of yellow sulfate crystals. According to the results of ion chromatographic analysis, the molar ratio of compound **1**/sulfate in the crystals was 1/1.0.

The resulting sulfate crystals of compound **1** were characterized by XRPD and the X-ray powder diffractograms are shown in Fig. 12, with characteristic peaks at diffraction angles (20 ± 0.20°) of 8.355°, 13.896°, 16.727°, 17.387°, 17.777°, 20.225°, 20.621°, 21.807°, 22.775°, 24.194°, 25.196°, 25.991°, 27.210°, 27.983°, 29.643°, 31.083°, 31.926°, 33.803°, 34.742°, 35.763°, and 38.545°. This crystal is defined as polymorph F of sulfate salt. The resolved data of the XRPD pattern of polymorph F of sulfate salt of compound **1** is shown in Table 9 below.

**Table 9**

| No | Diffraction angle (2θ) [°] | Relative strength (%) | Intensity |
|---|---|---|---|
| 1 | 8.355° | 100.0% | 893.4740 |
| 2 | 13.896° | 44.4% | 403.464 |
| 3 | 16.727° | 4.7% | 62.6590 |
| 4 | 17.387° | 2.0% | 41.7922 |
| 5 | 17.777° | 19.1% | 186.361 |
| 6 | 20.225° | 5.4% | 71.7389 |
| 7 | 20.621° | 14.5% | 147.6050 |
| 8 | 21.807° | 10.6% | 112.773 |
| 9 | 22.775° | 7.4% | 84.0322 |
| 10 | 24.194° | 23.4% | 221.2580 |
| 11 | 25.196° | 31.3% | 291.0020 |
| 12 | 25.991° | 9.0% | 101.520 |
| 13 | 27.210° | 1.7% | 37.2443 |
| 14 | 27.983° | 29.1% | 267.3790 |
| 15 | 29.643° | 7.1% | 79.317 |
| 16 | 31.083° | 2.7% | 40.4177 |
| 17 | 31.926° | 9.7% | 102.345 |
| 18 | 33.803° | 7.2% | 78.198 |
| 19 | 34.742° | 1.3% | 27.8393 |
| 20 | 35.763° | 1.5% | 28.406 |
| 21 | 38.545° | 2.8% | 38.9828 |

The crystals were analyzed as DMSO solvates of polymorph F of sulfate salt of compound **1.**

### Example 12 Preparation of polymorph G of sulfate salt of compound 1

In a 10 mL vial, 10 mg of polymorph A of sulfate salt prepared according to Example 1 and 0.8 mL of DMSO were added, slowly stirred until completely dissolved. The solution was filtered, and 6 volumes of deionized water was slowly added to the filtrate until a sufficient amount of solids precipitated. Filtration was performed, and the filter cake was vacuum dried for 2 hours at 40 °C to obtain 9 mg of yellow sulfate crystals. According to the results of ion chromatographic analysis, the molar ratio of compound **1**/sulfate in the crystal was 1/1.0.

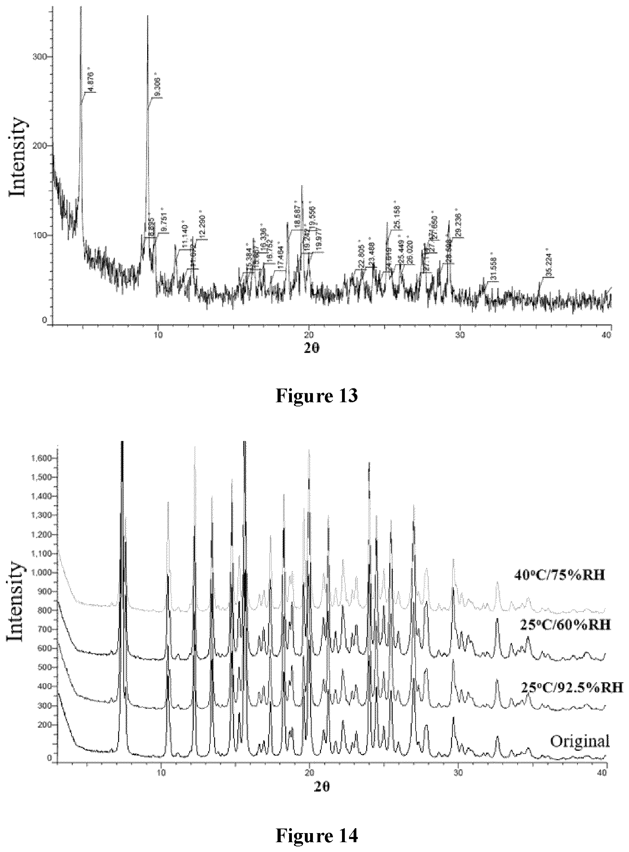

The resulting sulfate crystals of compound **1** were characterized by XRPD and the X-ray powder diffractograms are shown in Fig. 13, with characteristic peaks at diffraction angles (2θ ± 0.20°) of 4.876°, 8.895°, 9.306°, 9.751°, 11.140°, 11.672°, 12.290°, 15.384°, 15.867°, 16.336°, 16.752°, 17.464°, 18.587°, 19.242°, 19.556°, 19.977°, 22.805°, 23.488°, 24.619°, 25.158°, 25.449°, 26.020°, 27.471°, 27.650°, 28.598°, 29.236°, 31.558°, 35.224° and 39.656°. This crystal is defined as polymorph G of sulfate salt. The analyzed data of the XRPD pattern of polymorph G of sulfate salt of compound **1** is shown in Table 10 below.

**Table 10**

| No | Diffraction angle (2θ) [°] | Relative strength (%) | Intensity |
|---|---|---|---|
| 1 | 4.876° | 80.8% | 245.918 |
| 2 | 8.895° | 18.9% | 83.5553 |
| 3 | 9.306° | 100.0% | 240.152 |
| 4 | 9.751° | 24.2% | 89.2217 |
| 5 | 11.140° | 15.5% | 70.1753 |
| 6 | 11.672° | 5.6% | 48.8656 |
| 7 | 12.290° | 23.9% | 81.2167 |
| 8 | 15.384° | 6.3% | 44.5163 |
| 9 | 15.867° | 7.8% | 47.6423 |
| 10 | 16.336° | 17.3% | 66.9107 |
| 11 | 16.752° | 10.5% | 54.3919 |
| 12 | 17.464° | 6.6% | 46.6815 |
| 13 | 18.587° | 30.7% | 92.374 |
| 14 | 19.242° | 16.4% | 66.1767 |
| 15 | 19.556° | 29.3% | 91.5357 |
| 16 | 19.977° | 17.1% | 67.4772 |
| 17 | 22.805° | 9.9% | 48.2497 |
| 18 | 23.488° | 10.3% | 50.895 |
| 19 | 24.619° | 5.9% | 44.958 |
| 20 | 25.158° | 29.4% | 91.9766 |
| 21 | 25.449° | 9.6% | 53.5236 |
| 22 | 26.020° | 9.6% | 53.034 |
| 23 | 27.471° | 17.7% | 66.6337 |
| 24 | 27.650° | 24.8% | 80.5577 |
| 25 | 28.598° | 12.3% | 54.6088 |
| 26 | 29.236° | 27.3% | 83.421 |
| 27 | 31.558° | 6.3% | 34.5313 |
| 28 | 35.224° | 8.8% | 38.9144 |
| 29 | 39.656° | 7.7% | 33.9369 |

The crystals were analyzed as DMSO solvate compounds of polymorph G of sulfate salt of compound 1.

Although the sulfate salt of compound **1** can be obtained in a variety of crystals (polymorph A, B, C, D, E, F and G: wherein polymorph A is a hydrate, polymorph B and polymorph C are acetonitrile solvate compounds; polymorph D is a DMF solvate compound; and polymorph E, F and G are DMSO solvate compounds), and the polymorph C, D, E, F and G are all prepared by slurrying the polymorph A as raw material in the corresponding solvents or by adding anti-solvents. However, the inventor found that the polymorph B, C, D, E, F and G of sulfate salt of compound **1** are converted to polymorph A after each of them has been placed under atmospheric conditions for one week. Therefore, polymorph A of sulfate salt of compound **1** is an advantageous crystal form among the many sulfate crystal forms, and it has better storage stability. The subsequent experiments were carried out for polymorph A of sulfate salt of compound **1.**

### Example 13 Stability evaluation of polymorph I of Compound 1 and its sulfate polymorph A

10 mg polymorph I of compound **1** obtained in Comparative Example 1 and polymorph A of sulfate salt obtained in Example 1 were placed in an airtight environment and stored at 2~8 °C, 25 °C and 60 °C for 1 week and 2 weeks, respectively, and then samples were taken to examine the chemical purity of the samples by HPLC and the crystal morphology of the samples by XRPD. The results are shown in Table 11.

**Table 11 Evaluation of storage properties of crystals at different temperatures**

| | | polymorph I of Compound **1** | | Sulfate polymorph A | |
|---|---|---|---|---|---|
| | | Purity (%) | the amount of aromatic hydrocarbons (%) | Purity (%) | the amount of aromatic hydrocarbons (%) |
| Initial | | 97.4 | 1.6 | 97.5 | 1.5 |
| One week | 2~8 °C | 97.6 | 1.4 | 97.5 | 1.5 |
| | 25 °C | 97.6 | 1.4 | 97.4 | 1.5 |
| | 60 °C | 96.6 | 2.4 | 97.3 | 1.7 |
| Two weeks | 2~8 °C | 97.5 | 1.5 | 97.5 | 1.5 |
| | 25 °C | 97.4 | 1.6 | 97.5 | 1.5 |
| | 60 °C | 96.3 | 2.7 | 97.1 | 1.8 |

The characterization results of the crystal structure showed that the polymorph I of compound **1** did not change significantly after storage for 2 weeks at low temperature (2-8 °C) or room temperature (25 °C). However, when stored at high temperature (60 °C) for 2 weeks, the crystal structure changed and the purity of the product was reduced to a certain extent. This indicates that the stability of the substance in the above-mentioned environments, especially at high temperature, needs to be improved.

The characterization results of the crystal structure showed that the polymorph A of sulfate salt of compound **1** did not undergo significant changes after storage for 1 week and 2 weeks at 2~8 °C, 25 °C and 60 °C, respectively, and the chemical purity of the polymorph A hardly decrease significantly. This indicates that compound **1** can remain stable under different temperature conditions for at least 2 weeks after forming sulfate crystals.

Therefore, it can be concluded that compound **1** exhibits good physical and chemical stability after the formation of sulfate crystals, which obviously improves the structural stability of the original compound **1** and is conducive to the stable preservation of the drug prepared from this compound and its salt forms.

### Example 14 Evaluation of the temperature and humidity stability of sulfate polymorph A

The sulfate polymorph A obtained in Example 1 was placed openly under conditions of 25°C/92.5% RH, 25°C/60% RH and 40°C/75% RH to test the temperature and humidity stability. After the samples were stored for 1 week and 4 weeks, respectively, the purity of the samples was tested by HPLC, and the crystalline morphology of the samples was detected by XRPD. The results are listed in Table 12.

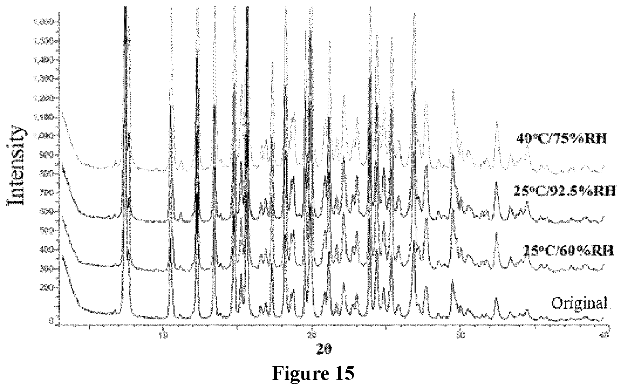

The XRPD results of sulfate polymorph A after 1 and 4 weeks of storage at 25°C/92.5% RH, 25°C/60% RH and 40°C/75% RH are shown in Figures 14 and 15.

**Table 12 Stability assessment of sulfate polymorph A at different temperatures and humidities**

| | Sulfate polymorph A | |
|---|---|---|
| | Purity (%) | Aromatic content (%) |
| Initial | 98.1 | 1.0 |
| 25°C/92.5% RH for one week | 98.1 | 1.0 |
| 25°C/60% RH for one week | 98.1 | 1.0 |
| 40°C/75% RH for one week | 98.1 | 1.0 |
| 25°C/92.5% RH for four weeks | 97.8 | 1.2 |
| 25°C/60%RH for four weeks | 98.1 | 1.0 |
| 40°C/75% RH for four weeks | 97.9 | 1.1 |

As shown in Table 12, Fig. 14 and Fig. 15, the polymorph A of sulfate salt of compound **1** exhibits high structural stability and can be stably stored under different humidity and temperature conditions.

### Example 15 Moisture adsorption-desorption experiments with sulfate polymorph A

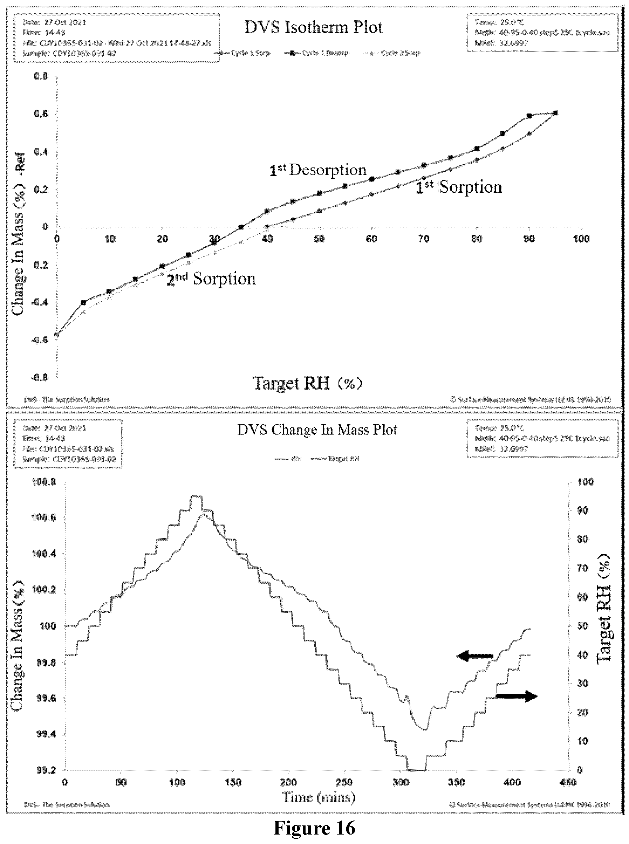

The moisture adsorption-desorption properties of sulfate polymorph A obtained according to Example 1 were examined by DVS. 16 mg of sulfate polymorph A was placed in a metal container, which was placed in an Intrinsic DVS apparatus. A cyclic humidity gradient of 40-95-0-40% RH was set at 25 °C, and the samples were subjected to two successive adsorption-desorption cycles. At each stage, the following equilibrium criteria were used: mass change dm/dt < 0.002% over 5 min, with minimum and maximum equilibrium times of 60 min and 360 min, respectively, at each stage. The results are shown in Table 13 and Figure 16.

**Table 13 Dynamic water adsorption experiments for sulfate polymorph A**

| Relative humidity (%) | 1st Adsorption Mass change (%) | 1st Desorption Mass change (%) | 2nd Adsorption Mass change (%) |
|---|---|---|---|
| 0 | - | -0.574 | -0.574 |
| 10 | - | -0.344 | -0.368 |
| 20 | - | -0.210 | -0.246 |
| 30 | - | -0.084 | -0.133 |
| 40 | 0.000 | 0.081 | -0.017 |
| 50 | 0.085 | 0.178 | - |
| 60 | 0.174 | 0.253 | - |
| 70 | 0.260 | 0.327 | - |
| 80 | 0.357 | 0.418 | - |
| 90 | 0.497 | 0.589 | - |
| 95 | 0.604 | 0.604 | - |

From the results of the hygroscopicity test, it can be seen that under the condition of 25 °C, the sulfate polymorph A absorbed 1.2% of moisture in the range of 0%RH-95%RH, so the sulfate polymorph A is slightly hygroscopic. This indicates that when the compound exists in the form of sulfate polymorph A, it can remain stable without strict humidity control during the production and storage of the drug. The requirements for the preparation process and storage conditions are reduced.

### Example 16 Solubility experiment

Polymorph I of compound **1** obtained in Comparative Example 1, and sulfate polymorph A obtained in Example 1 were weighed in a sample vial, to which 1 mL of hydrochloric acid buffer solution at pH=1.2, acetate buffer solution at pH=4.5, phosphate buffer solution at pH=6.8, and water were added, respectively. The suspension was magnetically stirred at 25°C (1000 r/min). Samples were taken at 24 h, centrifuged, and the supernatants were collected for solubility detection by HPLC. The results of solubility assessment of compound **1** and its sulfate polymorph A are summarized in Table 14.

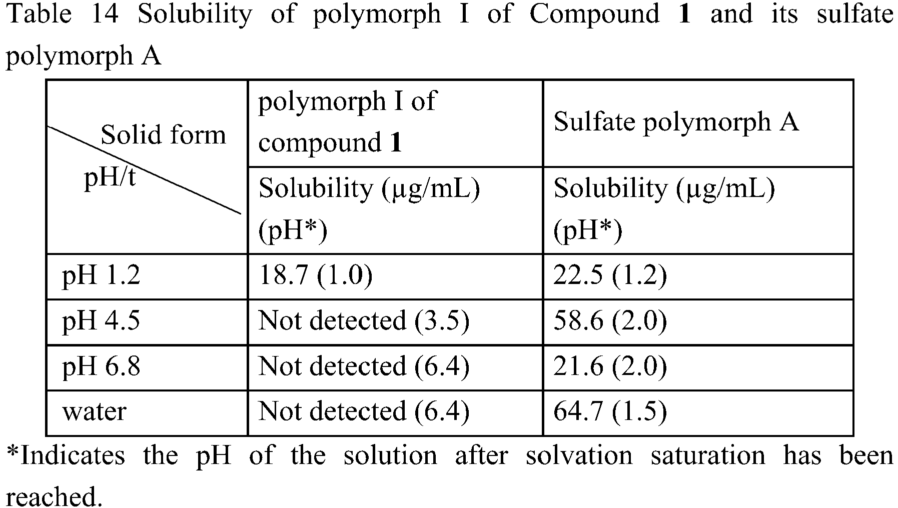

From the above table, it can be seen that the solubility of sulfate polymorph A in acetate buffer solution at pH=4.5, phosphate buffer solution at pH=6.8 and water can be significantly increased, which improves the drugability of the compound, and is conducive to the increase of the concentration of the drug *in vivo,* thus facilitating drug absorption.

### Industrial Applicability

The present invention provides a salt of a MNK inhibitor, in particular its sulfate salt and crystalline polymorphs, along with a preparation method. The prepared salt of the compound can be applied as a MNK inhibitor, which has potential therapeutic effects in the treatment of various diseases such as proliferative disorders, inflammatory diseases, and neurodegenerative diseases. Therefore, the present invention is suitable for industrial application.

It should be noted that the present application is not limited to the above embodiments. The above-described embodiments are only examples, and any embodiments within the scope of the technical solution of the present application that have configurations substantially consistent with the technical concept and performing the same effects are included in the technical scope of the present application. Furthermore, without departing from the essence of the present application, any modifications conceivable by those skilled in the art made to the embodiments, as well as other modes constructed by combining some of the constituent elements of the embodiments, are also included within the scope of the present application.

## Claims

1. A salt of a compound of Chemical Formula 1, **characterized in that**: 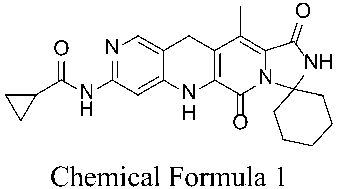
the salt comprises a free base moiety and an acid moiety;
the free base moiety has the structure of Chemical Formula 1;
the acid moiety is any one selected from hydrochloric acid, sulfuric acid, methanesulfonic acid or formic acid.

2. The salt according to claim 1, **characterized in that**: the salt is in a crystalline form.

3. The salt according to claim 1 or 2, **characterized in that**: the acid moiety is sulfuric acid.

4. A crystal of sulfate salt of a compound of Chemical Formula 1, **characterized in that**: 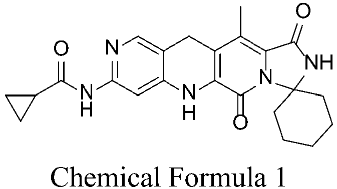 the crystal has a crystal structure of polymorph A, the X-ray powder diffraction pattern of the polymorph A exhibits characteristic peaks at 2θ values of 7.44±0.2°, 15.72±0.2°, 20.05±0.2°, 24.10±0.2°, 24.57±0.2°, 25.55±0.2° and 27.10±0.2°.

5. The crystal according to claim 4, **characterized in that** the X-ray powder diffraction pattern of the polymorph A further exhibits one or more characteristic peaks at 2θ values selected from: 12.33±0.2°, 13.50±0.2°, 14.83±0.2°, 18.33±0.2°, 19.70±0.2°, 21.30±0.2° and 29.77±0.2°.

6. The crystal according to claim 4, **characterized in that**: the X-ray powder diffraction pattern of the polymorph A exhibits characteristic peaks at 2θ values of 7.44±0.2° , 10.52±0.2° , 12.33±0.2° , 13.50±0.2° , 14.83±0.2° , 15.72±0.2° , 17.43±0.2° , 18.33±0.2° , 19.70±0.2° , 20.05±0.2° , 21.30±0.2° , 24.10±0.2° , 24.57±0.2° , 25.55±0.2° , 27.10±0.2° , 27.93±0.2° , and 29.77±0.2° .

7. The crystal according to claim 4, **characterized in that**: the X-ray powder diffraction pattern of the polymorph A exhibits characteristic peaks at 2θ values of 7.44±0.2° , 10.52±0.2° , 11.23±0.2° , 12.33±0.2° , 13.50±0.2° , 14.83±0.2° , 15.34±0.2° , 15.72±0.2° , 17.43±0.2° , 18.33±0.2° , 18.81±0.2° , 19.70±0.2° , 20.05±0.2° , 21.30±0.2° , 22.31±0.2° , 23.20±0.2° , 24.10±0.2° , 24.57±0.2° , 25.05±0.2° , 25.55+0.2 ° , 26.04±0.2° , 27.10±0.2° , 27.93±0.2° , 29.77±0.2° , 30.33±0.2° , 30.99±0.2° , 32.10±0.2° , 32.75±0.2° , 33.69±0.2° , 34.83±0.2° , and 35.83±0.2° .

8. The crystal according to any one of claims 4 to 7, **characterized in that**: the crystal of sulfate salt is a hydrate.

9. A method of preparing the crystal of any one of claims 4 to 8, **characterized in that**:
mixing the compound of Chemical Formula 1 with a solvent selected from water, alcohols, ketones or mixtures thereof, and forming a suspension after the addition of sulfuric acid solution; stirring and then isolating the suspension to obtain the crystal.

10. The method according to claim 9, **characterized in that**, the solvent is a mixture of water and ketones.

11. The method according to claim 9, **characterized in that**, the solvent is a mixture of water and acetone.

12. The method according to claim 9, **characterized in that**, adding 1.1 to 2.1 equivalents of sulfuric acid solution.

13. A method of preparing the crystal of any one of claims 4 to 8, **characterized in that**:
mixing the compound of Chemical Formula 1 with a solvent selected from water, alcohols, ketones or mixtures thereof, and forming a suspension after the addition of sulfuric acid solution; adding the polymorph A of sulfate salt of the compound of Chemical Formula 1 to the suspension as a seed crystal; stirring and then isolating the suspension to obtain the crystal.

14. The method according to claim 13, **characterized in that**, the solvent is selected from water, methanol, ethanol, n-propanol, isopropanol, 1-butanol, tert-butanol, acetone, butanone, 2-pentanone or mixtures thereof.

15. A pharmaceutical composition comprising the salt of any one of claims 1 to 3 or the crystal of any one of claims 4 to 8 and a pharmaceutically acceptable excipient.
